# EUROPEAN PATENT APPLICATION

(11) **EP 1 767 515 A1**
(43) Date of publication of application: **28.03.2007**
(21) Application number: 05774304.9
(22) Date of filing: 09.06.2005
(51) Int. Cl.: C07C 68/00, C07C 69/96

(54) **METHOD OF OBTAINING POLYOXYGENATED ORGANIC COMPOUNDS**

(30) Priority: 11.06.2004 ES 200401508
(71) Applicant: CONSEJO SUPERIOR DE INVESTIGACIONES CIENT FICAS, 28006 Madrid (ES); UNIVERSIDAD POLITECNICA DE VALENCIA, 46022 Valencia (ES)
(72) Inventor: CORMA CANOS, Avelino, Inst. de Tecnología Química, 46022 VALENCIA (ES); DOMINE, Marcelo E., Inst. de Tecnología Quimica, 46022 VALENCIA (ES); RENZ, Michael, Inst. de Tecnología Química, 46022 VALENCIA (ES)
(74) Representative: Ungria Lopez, Javier
(86) International application number: PCT/ES2005/070088
(87) International publication number: WO 2005/123654

(57) **Abstract**

The invention relates to a method of obtaining polyoxygenated organic compounds. The inventive method is **characterized in that** it comprises the oxidation reaction of a diether, preferably an acetal, with an oxygen source, in the presence of: one or more radical initiating agents, one or more additives that generate a basic reaction medium, and one or more catalysts.

## Description

### FIELD OF THE ART

Homogenous catalysis, heterogeneous catalysis, petrochemistry.

### BACKGROUND OF THE INVENTION

Different types of oxygenated organic compounds, and particularly, polyoxygenated organic compounds containing from 3 to 12 carbon atoms, and more specifically from 3 to 5 carbon atoms, and 3 or more oxygen atoms, are widely used in different types of industries, such as the plastics and pharmaceutical industries, and also as fuel additives in the refining industry.

Organic carbonates, very interesting and versatile intermediates of the synthesis process, are amongst these oxygenated compounds. They are used in the preparation of lubricants, solvents and plastifying agents, and especially as monomers for obtaining the different polycarbonates. For instance, diphenyl carbonates (DPC) or dimethyl carbonates (DMC) obtained by oxidative carbonylation using CO and O₂ [J. I. Kroscwitz et al. (eds.), Kirk-Othmer Encyclopedia of Chemical Technology, 4th ed., Vol. 19, J. Wiley & Sons, New York, 1996, p. 584] are widely used in the preparation of different polycarbonates, that can also be obtained by processes that involve phosgene (COCl₂), a dangerous chemical compound that is toxic to the environment [C.D. Chang et al., US-6008399, 1999].

Other alternative, less dangerous and polluting methods have been proposed for carbonate synthesis, to substitute phosgene as a reactive in the process. One of these methods entails the catalytic reaction of methanol with CO and O₂ to form dimethyl carbonate, using a Cu salt as catalyst. In this case, the low selectivities obtained and the partial dilution of the metal salt in the reactive medium are the main disadvantages [J. E. Hallgren et al., US-4360477, 1982]. In this sense, Anic S. p. A. [U. Romano et al., US-4318862, 1982; and US-4218391, 1972] has softened the negative effect of the non dissolved copper salt by working with excess methanol in the process, although it translates in a lower dimethyl carbonate performance and the subsequent and complicated separation of the carbonate and the water generated during the process.

Other methods of obtaining carbonates use transition metal complexes in homogenous phase [Z. Kricsfalussy et al., EP-659731, 1995]. In this line, the use of these Me complex catalysts have been proposed. They contain transition metals capable of changing their oxidation state by oxyreduction reactions throughout the process, with an aliphatic alcohol, CO and O₂ to obtain the corresponding organic carbonate [Gaenzler, W., US-3962045. 1975]. In all said cases, the impossibility of recovering said catalysts for reuse and the lack of economic viability of these processes are added to the inherent difficulties of the making and handling of catalysts.
On the other hand, there are several existing types of processes that can be used to produce organic carbonates in an accessible manner, such as: the reaction of urea or urethane with alcohols and a catalyst, the reaction of alkyl halides or alkyl sulphates with alkaline carbonates, the reaction of CO with alkyl nitriles and a catalyst and the reaction of olefins with ozone, although all these processes have scant significance at the industrial level.
In 1999, C. D. Chang et al. [US-6008399] claimed the use of an organic acetal (dimethoxymethane, DMM), a source of oxygen and a catalyst based on a Ru complex used to obtain an organic carbonate (dimethyl carbonate, DMC). In this case to the need of having to prepare a catalyst solution in situ, the low carbonate yields are added as disadvantages.
Organic carbons, in this case cyclic, can also be obtained from a reaction of an organic epoxide with CO₂, by a cycloaddition process catalyzed by acids [H. Matsuda et al., Chem. Lett., 1979, 573; R. Nomura et al., J. Org. Chem., 1980, 3735]. This type of reactions involve transition metal complexes catalysts, such as alkyl Zn or alkyl Al, dialkyl methoxy stannates, organic antimony halides, which are toxic and water and air sensitive, which causes operational problems, as well as requiring high reaction pressures and temperatures to reach acceptable levels of conversion and selectivity.

The use of other types of catalysts for this process has been published in papers such as alkaline metal salts [K. Nihara et al., J. Org. Chem., 58, 1993, 6198], mixed Al and Mg oxides [K. Yamaguchi, et al., J. Am. Chem. Soc., 121, 1999, 4526], metal complexes with Schiff bases as ligands [X. -B. Lu et al., Appl. Catal. A: Gen., 234, 2002, 25], metal porphyrines [W. J. Rupers et al., J. Org. Chem., 60, 1995, 725] and phtalocyanines [X. -B. Lu et al., J. Mol. Catl., 186, 2002, 33], where the cost of the catalysts and the difficulties of separating them from the reaction media present additional complications. A solution to these constraints is the heterogenization of these metal catalysts by encapsulating metal phtalocyanines in zeolitic matrixes, such as Y zeolite [Srivastava et al., Catal. Lett., 89, 2003, 81]. Other type of heterogeneous catalysts have been assayed, such as TS-1 and Ti-MCM-41 [Srivastava et al., Catal. Lett., 91, 2003, 81], in order to generate from the olefin epoxide the corresponding diol, that at a later stage reacts with CO₂ to yield the corresponding cyclic organic carbonate with very good selectivities, although with very low conversions.

Also, in the last few years the interest in the use of polyoxygonated compounds as additives in fractions of liquid fuels has increased in order to increase the burning process of said fuels in the engines, and especially to increase their combustion during the vapor phase, in order to enhance the fuel's performance and decrease atmospheric pollution by decreasing the production of toxic gases (COx, NOx) generated during the partial combustion of the hydrocarbons present in the fuels [W. C Orr, WO-9966009, 1999]. These compounds, generically called ECS "enhanced combustion structure" [WO-9966009] are added in proportions that go from 0.01 to 5% by weight in gasolines, where oxygen content is essential and represents at least between 10 and 40% by weight of the structure. Examples of this type of compounds are different organic carbonates and other polyoxygenated compounds such as: ethers, diethers, esters, diesters, hydroxyethers, hydrosyesters, glycols, alcohols, aldehydes, ketones, carboxyalcohols, alkoxy aldehydes, alkoxy ketones, amongst many others.

It seems evident, from the information described above, the need to develop catalytic alternative processes to produce polyoxygenated organic compounds such as, amongst others, organic carbonates in order to avoid the use of dangerous and polluting reactives, and that are at the same time economically and industrially viable and cost-effective.

### DESCRIPTION OF THE INVENTION

The present invention refers to a procedure to obtain polyoxygenated organic compounds characterized in that it comprises an oxidation reaction with a diether and a source of oxygen acting as oxidating agent in the presence of:
one or more radical initiating agents
   - one or more additives that generate a basic reaction medium, and
   - one or more catalysts.

According to one embodiment of the present invention, polyoxygenated compounds are preferably organic carbonates, alkoxy ethers, alkoxy diethers, alkoxy esters or alkoxy diesters. Examples of diethers are, amongst other and without being limited to the examples provided: 1,3 -dioxane; 1,3, dioxolane; 1,4-dioxane; 1, 4-dioxolane and trioxane.
According to a particular embodiment of the invention, the organic carbonate type polyoxygenated organic compounds may respond to the general formula:

R₁-O-CO-O-R₂

where R₁ and R₂ are the same or different substituents and have been indistinctively selected between an alkyl with 1 to 12 carbon atoms, linear or branched, substituted or non-substituted; cyclic alkyl with 4 to 12 carbon atoms, substituted or non-substituted; and an aryl with 6 to 18 carbon atoms, substituted or non-substituted. Examples of these compounds are: dimethyl carbonate, diethyl carbonate, di-n-propyl carbonate, di-iso-propyl carbonate, di-n-butyl carbonate, di-iso-butyl carbonate, di-sec-butyl carbonate, di-2-ethyl-hexyl carbonate, di-cyclohexyl carbonate and di-fenyl carbonate.
According to an additional particular embodiment of the present invention, polyoxygenated organic compounds have the following formula:

R₁-CO-O-CH₂-O-R₂

where R₁ and R₂ are the same or different substituents and have been indistinctively selected between an alkyl with 1 to 12 carbon atoms, linear or branched, substituted or non-substituted; cyclic alkyl with 4 to 12 carbon atoms, substituted or non-substituted; and an aryl with 6 to 18 carbon atoms, substituted or non-substituted.
The procedure described in the present invention can also be used to obtain organic polyoxygenated compounds with the following general formula:

R₁-CO-O-CH₂-O-CO-R₂

where R₁ and R₂, are the same or different substituents and have been indistinctively selected between an alkyl with 1 to 12 carbon atoms, linear or branched, substituted or non-substituted; cyclic alkyl with 4 to 12 carbon atoms, substituted or non-substituted; or an aryl with 6 to 18 carbon atoms, substituted or non-substituted.

In all cases mentioned above, the polyoxygenated organic compounds obtained from the oxidation of an organic acetal are characterized in that their structure possess a preferred percentage by weight of oxygen between 20 and 70%, and preferably between 30 and 60%. Preferably, the C/O rate in the structure should be less than 2.

According to the procedure object of the present invention, an example of diether is 1,4-dioxane, 1,4-dioxolane or an acetal. The diether is preferably an acetal. Said organic acetal is selected, preferably, between one or more linear aliphatic acetals, branched aliphatic acetals, cyclic acetals, aromatic acetals and combinations thereof.
The initial organic acetal used in the procedure object of the present invention may be selected between compounds that respond to the following generic formula:

R₁-O-CH₂-O-R₂

where R₁ and R₂ are the same or different substituents and have been indistinctively selected between an alkyl with 1 to 12 carbon atoms, linear or branched, substituted or non-substituted; cyclic alkyl with 4 to 12 carbon atoms, substituted or non-substituted, and an aryl with 6 to 18 carbon atoms, substituted or non-substituted.
Preferably, the organic acetal may be selected between di-methoxy-methane, di-ethoxy-methane, di-n-propoxy-methane, di-iso-propoxy-methane, di-n-butxy-methane, di-iso-butoxy-methane, di-sec-butoxy-methane, di-2-ethyl-hexoxy-methane, di-cyclohexoxy-methane, acetals derived from di-phenyl methane, amongst others, without these examples being a limitation of the options. Also, the organic acetal may be selected between 1,3-dioxane, 1,3-dioxolane and trioxane, amongst others, without said example being limiting themselves.

The oxidation reaction is done in the presence of an activating or free radical initiating agent, which function is to accelerate the onset of the reactive process, favoring the formation of the first free radicals in the reactive medium to initiate the radical chain.
The following can be used as free radicals initiator agent: organic nitriles, organic peroxides, inorganic peroxides, organic hydroperoxides and inorganic hydroperoxides, these examples offered as examples and not limitations.

Preferably, and in the case of compounds with functional groups such as nitrile groups, these compounds respond to the general formula of:

R₁-C ≡ N

where R₁ has been selected from an alkyl group with 1 to 12 carbon atoms, linear or branched, substituted or non-substituted; a cyclic alkyl with 4 to 12 carbon atoms, substituted or non-substituted; and an aryl group with 6 to 18 carbon atoms, substituted or non-substituted; an alkylazo with 1 to 12 carbon atoms, linear or branched, substituted or non-substituted; a bis-alkylazo with 1 to 12 carbon atoms, linear or branched, substituted or non-substituted; an arylazo group with 6 to 18 carbon atoms, substituted or non-substituted; a bis-arylazo group with 6 to 18 carbon atoms, substituted or non-substituted, plus preferably, the following organic nitriles: acetonitrile, butyronitrile, iso-butyronitrile, phenylnitrile, azobutyronitrile, azo-iso-butyronitrile, azobisisohutyronitrilc, azo-phenylnitrile, azobisphenylnitrile, these compounds offered as an illustrative but not limiting example.

When peroxide type compounds are used as initiators, organic peroxides like diperoxides, alkylic or aromatic organic compounds are preferred, such as dibenzoic peroxide, diphenyl peroxide, ditertbutyl peroxide, diperoxic compounds with the following general formula:

R₁- C- O -O- R₂

where R₁ and R₂ are the same or different substituents and selected from an alkyl with 1 to 20 carbon atoms, linear or branched, substituted or non-substituted; cyclic alkyl with 4 to 12 carbon atoms, substituted or non-substituted; and an aryl with 6 to 18 carbon atoms, substituted or non-substituted.

When hydroperoxide type compounds are used, organic peroxides such as: cumen hydroperoxide (CHP), ethyl-benzene-hydroperoxide (EBHP), tertbutyl hydroperoxide (TBHP) and generally those hydroperoxides that respond to the following general formula are preferred:

R₁-C-O-O-H

where R₁ represents a substituent selected between an alkyl with 1 to 20 carbon atoms, linear or branched, substituted or non-substituted; cyclic alkyl with 4 to 12 carbon atoms, substituted or non-substituted; and an aryl with 6 to 18 carbon atoms, substituted or non-substituted.

In the procedure involving the selective oxidation of diethers, and especially organic acetals, described in the present invention, the basic medium (pH between 9 and 12), may be obtained by adding and additive that may be one or more basic salts containing alkaline ions, alkali earths ions, transition metal ions, or mixtures of these compounds thereof. Said buffer salt or compound used to control pH levels, may be constituted by an organic or inorganic anion and a metal cation from the alkaline metal group, the alkaline earth group (groups Ia, IIa of the periodic table), or from the transition elements (such as from Ib, IIb, IVb, Vb, VIb, VIIb, and VIII groups of the periodic table) and combinations thereof.
In the case of salts composed by organic anions, these can be preferably selected from, formiates, acetates, butyrates, adipates, oxalates, phtalates, terephatalates, carbonates and combinations thereof, amongst many others.

In the case of salts composed by inorganic anions, these can be, for instance of the carbonate, phosphate, sulphate, nitrate, chlorate, bromate, phosphonate, sulphonate, phosphite, sulphite, nitrite, chlorite, bromite type, or combinations thereof.

According to the procedure object of the present invention, the source of oxygen may be selected from pure molecular oxygen, air, oxygen enriched air, a gaseous mix containing oxygen and combinations thereof of the previous components. Said gaseous mixture may comprise two or more gases.

In this manner, air, for instance, may be used as a source of oxygen, oxygen enriched ozone, oxygen enriched N₂, oxygen enriched Ar or mixtures thereof, without these examples being limiting. The amount of oxygen and the selected source will depend on the type of reactor used and the specific reaction conditions. Preferably, the quantity of oxygen present in the reactive medium will be close to the stoichiometric quantity or less (always in relation to the initial diether quantity used, particularly in the case of organic acetals), and depending on the temperature and pressure conditions in the reactor, in order to minimize undesired secondary reactions.

The catalyst used in the oxidation process object of the present invention may be a transition metal salt. Said salts operate in homogeneous or semi-homogenous phase (partially dissolved in the reactive mixture). Amongst them are the transition metals found in groups Ib, IIb, IVb, Vb, VIb, VIIb and VIII of the periodic table. Preferably, the metal catalysts will be Cu, Co, Ni, Fe, Mn, and Ce salts and combination of said elements thereof. The counterion of these salts may be selected from inorganic anions of the following type: halides, nitrites, nitrates, sulphites, sulphates, sulphonates, phosphates, phosphites, phosphonates, and also from amongst organic anions such as formiates, acetates, carbonates, oxalates, butyrates, adipates, phtalates, terphtalates, amongst other, without these examples being limited to the mentioned anions.

In a particular embodiment of the process object of the present invention, the organic acetal oxidation catalyst may be a metal complex containing one or more organic ligands coordinated, at least, to one transition metal. Said transition metal may be one or more from the Ib, IIb, IVb. Vb, VIb, VIIb and VIII groups of the periodic table. For instance said metal may be Cu, Co, Mn, Ni, Fe, Ce and combinations thereof. Preferably, the organic ligands may be selected between amines, alkylamines, dialkylamines, trialkylamines, diamines, pyridines, substituted pyridines, pyrrolidines, substituted pyrrolidines, bipyridines, imines, pyrrols, substituted pyrrols, imidiazoles, substituted imidiazoles, porphyrines, phtalocyanines, and combinations thereof, amongst other and without these examples being limiting.

In another particular case of the diether oxidation process, and more particularly, the organic acetals oxidation process object of the present invention, the metal catalyst may comprise a compound selected between a salt and a transition metal complex, said salt or complex supported or included in the structure of a solid or inorganic matrix, such as amorphous solids, or microporous molecular sieves, mesoporous molecular sieves and combinations thereof.
The following are non-limiting examples of amorphous solid matrixes used in the process: silica, alumina, ceria, silica-alumina, silica-ceria, and in general mixed metal oxides and/or transition metals such as Cu, Zn, Ti, Ce, Mn, V, Ni, Fe, Sn, Mo amongst other.
The following are non-limiting examples of microporous solid matrixes that may be used in the process: microporous silicates, including pure silica zeolites, microporous aluminum-silicates including Al-zeolites, microporous metal-silicates including Me-zeolites, microporous aluminum-phosphates (AIPOs, APOs, etc.), metal containing microporous aluminum-phosphates (Me-APOs), microporous silica-aluminum-phosphates (SAPOs, TAPSOs, etc.) and combinations thereof. Laminar materials such as clays and pillared clays such as bentonite, montmorillonite, amongst others, can also be used as microporous inorganic matrixes, as well as their combinations thereof.

As non-limiting examples of mesoporous solid matrixes used, the following can be mentioned: silicates, aluminosilicates, and in general mesoporous metal silicates with hexagonal or cubic structure such as MCM-41, MCM-48, SBA-15, HMS, MSA, amongst other. The following can also be used as mesoporous solid matrixes: mesoporous materials obtained by delamination of laminar zeolitic precursors such as ITQ-2 and ITQ-6, amongst other, and their combinations thereof.
The transition metal used in the supported catalysts may be a metal belonging to the following groups of the periodic table: Ib, IIb, IVb, Vb, VIb, VIIb and VIII, such as Cu, Co, Mn, Ni, Fe, Ce and combinations thereof.
The metal catalyst mentioned above may be ionically exchanged or coordinated to a polymeric matrix, for instance, an ionic exchange resin as amberlite or dawex, without these examples being limiting. The metal catalyst mentioned before can also be found coordinately linked to a polymeric matrix of the polyalcohol type, such as polyol, polyehtylenglycol, polyethylenglicol adipate; polyamides such as polypropylenamide, polyacrylamyde, poly-(dimmer co-alkyl-polyamine acid): polyamines, such as poly-alylamine, polycyclohexyl-diamine, poly-2-vynil-pyridine, poly-4-vynil-pyridine, poly-2-vynil-pyrrolidine, poly-4-vynil-pyrrolidine, polyphenylamine; polylactames such as polycaprolactame (nylon-6; 6/6, 6/9, 6/10, 6/12); polysulfones, such as poly-1-dodecene sulphone, polyphenylsulphone; without these examples being limiting.

In a particular embodiment of the procedure object of the present invention, the diether oxidation catalyst, especially the organic acetals, may be a solid configured by alkaline earth metal oxides (MgO, CaO, BaO), preferably MgO, with oxides of other types of metals, and in general mixed oxides derived from anionic clays, such as double laminar hydroxides of the hydrotalcite type (Mg/Al) having the following general formula:

[M^{II} ₁₋ₓ M^{III} ₓ(OH) ₂] [Aⁿ⁻]_{x/n} . zH₂O

in which:
Aⁿ⁻ is one or more charge compensation ions, and is decomposed at about 300-500 °C,
n is the negative charge of the compensation ion that can vary between n and 1 and 3,
M^{II} is one or more divalent metals, for instance Mn⁺², Ni⁺², Zn⁺², Fe⁺², Cu⁺², Co⁺², Ce⁺², amongst others,
M^{III} is a trivalent metal such as Al⁺³, Fe⁺³, Cr⁺³ amongst others,
z may vary between 1 and 10,

The charge compensation anion decomposes at around 300° -500° C in a volatile gas,
When these clays are calcined at between 300 and 700° C the result is an M⁺² and M⁺³ mixed oxide.
The charge compensation anion may be selected between combinations of one or more mono-, di-, tri- valent anions and combinations of same.
The preparation of the hydrotalcites that contain different transition metals beside Mg and Al, is done from gels obtained from dilutions of Mg (NO₃)₂, Al(NO₃)₂, M^{ll} (NO₃)₂, where M may be Cu, Co Ce, Ni, Zn, Fe, amongst others, with Na₂CO₃ and NaOH in perfectly well known concentrations and determined according to Corma et al., J. Catal, 148, 1994, 205; and J. Catal., 221, 2004, 62.

The process to carry out the selective oxidation of diethers, especially of organic acetals, is characterized in that the oxidation reaction may be carried out in a discontinuous reactor, a continuous stirred-tank reactor (CSTR), a continuous fixed bed reactor, a fluidized bed reactor or an ebullient bed reactor.

In an embodiment of the present invention, the diether selective oxidation procedure, especially of organic acetals, is done by putting in contact a reactive mixture containing a diether, especially a selected organic acetal, a source of oxygen (preferably O₂ or air), an activating agent, an alkali metal salt, alkali earth salt or a transition metal salt used to alkalinize the medium, with a homogenous metal catalyst, semi-homogeneous metal catalyst, or solid material that contains metallic species, or a mixture thereof, at a sequence of pressure intervals that can range from atmospheric pressure to 100 bars, set at 10 to 250 °C during reaction times that can vary between 2 minutes and 72 hours, depending on the catalyst used and on the reaction conditions.
In the procedure according to the present invention, the relation by weight of the diether, preferably organic acetal, to the catalyst is, preferably, between 1 and 1000, and preferably between 10 and 500.
The relation by weight of the diether, preferably organic acetal, to the oxidating agent may be, preferably, between 3 and 600, while the relation by weight of the diether, preferably organic acetal, to the initiating agent is, preferably, between 1 and 500, and the relation by weight of the diether, preferably organic acetal, to the additive required to generate an alkaline medium is, preferably, between 1 and 500.
If the oxidation process is done in a discontinuous reactor, the relation by weight of the diether, preferably organic acetal, to the catalyst is, preferably between 5 and 1000, and more preferably between 20 and 500. In the case of using a discontinuous reactor, the relation by weight of the diether, preferably organic acetal, to the oxidating agent is between 3 and 600. The reaction temperature for the procedure inside a discontinuous reactor is set preferably between 10 and 250° C, more preferably between 40 and 150° C. The reaction time in a discontinuous reactor is, preferably between 2 minutes and 72 hours. When the reaction is done inside a discontinuous reactor, the pH must be, preferably, between 7 and 12. The oxidation reaction, when done in a discontinuous reactor is done at a total system pressure between, preferably, atmospheric pressure and 100 bars.
When the oxidation reaction is done in a discontinuous reactor, the relation by weight of the diether, preferably organic acetal, to the initiating agent is preferably between 1 and 500.
When the oxidation reaction is done in a discontinuous reactor, the relation by weight of the diether, preferably organic acetal, to the basic salt is preferably between 1 and 500.
According to the procedure object of the present invention, the water generated during the oxidation reaction is separated at a stage in which either membrane filters, addition of specific adsorbents and addition of compounds capable to react with the water generated during the reaction can be used.

The present invention describes a procedure to prepare polyoxygenated compounds, amongst them, organic carbonates such as dialkyl carbonates, diaryl carbonates and cyclic carbonates amongst others, and generally, polyoxygenated organic compounds that have between 3 and 24 carbon atoms in their structure, and more specifically between 3 and 12 carbon atoms with at least 3 or more oxygen atoms.
By means of the procedure object of the present invention, excellent conversion and selectivity levels can be achieved towards the obtention of organic carbonates and different types of polyoxygenated organic compounds derived from formaldehyde acetals, aliphatic as well as cyclic, through the selective oxidation of organic acetals with O₂ or air, or mixtures of oxygen enriched gases, using an activation agent (radical initiating agent), a compound that generales a basic reaction medium and in the presence of a metal catalyst, in mild or moderate reaction conditions (temperature and pressure).
The following examples illustrate the preparation of the metal catalysts and their application during the selective oxidation reaction of organic acetals with O₂ or air to obtain various polyoxygenated organic products, organic carbonates amongst them.

### EXAMPLES

### Example 1: Preparation of a Cu containing Hydrotalcite material. Cu-hydrotalcite [Cu-HT].

The material known as Cu-hydrotalcite [Cu-HT] (Cu/Mg/AI) is prepared by a standard co-precipitation method using two dilutions. The first dilution contains Mg (NO₃)₂ 6H₂O, Al(NO₃)₂ 9H₂O, and Cu(NO₃)₂ 5H₂O, with a molar relationship of (Al + Mg + Cu) of 1.5. The second solution contains NaOH and Na₂CO₃ in concentrations sufficient to obtain the total precipitation of aluminum, magnesium and copper in the first dilution and to fix the pH at a value of 13. Both solutions are added slowly (addition velocity = 30 ml/hour) to the same recipient while maintaining a vigorous stirring action during 4 hours. The gel thus obtained is placed in an autoclave at a self-generated pressure of 60° C during 14 hours. Later the solid is recovered by filtration, and is washed with distilled water until its pH reaches 7 and the carbonate is no longer detected in the filtering process. The hydrotalcite thus obtained is calcined at 550° C in air atmosphere during 9 hours. The result is a mixed Cu/Mg/Al oxide. The final quantity of Cu and the Mg/Al ratio in the solid are determined by atomic absorption measurements. The percentage by weight of the Cu obtained for the Cu-hydrotalcite material pertaining to this example is 1.5.

### Example 2: Preparation of a Co containing Hydrotalcite material. Co-hydrotalcite [Co-HT].

The material known as Co-hydrotalcite [Co-HT] (Co/Mg/Al) is prepared by a standard co-precipitation method using two dilutions. The first dilution contains Mg (NO₃)₂ 6H₂O, Al(NO₃)₃ 9H₂O, and Co(NO₃)₂ 4H₂O, with a molar relationship of (Al + Mg + Co) of 1.5. The second solution contains NaOH and Na₂CO₃ in concentrations sufficient to obtain the total precipitation of aluminum, magnesium and cobalt in the first dilution and to fix the pH at a value of 13. Both solutions are added slowly (addition velocity = 30 ml/hour) to the same recipient while maintaining a vigorous stirring action during 4 hours. The gel thus obtained is placed in an autoclave at a self-generated pressure of 60° C during 14 hours. Later the solid is recovered by filtration, and is washed with distilled water until pH reaches 7 and the carbonate is no longer detected in the filtering process. The hydrotalcite thus obtained is calcined at 550° C in air atmosphere during 9 hours. The result is a mixed Co/Mg/Al oxide. The final quantity of Co and the Mg/Al ratio in the solid are determined by atomic absorption measurements. The percentage by weight of the Co obtained for the Co-hydrotalcite material pertaining to this example is 1.5.

### Example 3: Preparation of a Cu and Ce containing Hydrotalcite material. Cu- Ce-hydrotalcite [Cu-Ce-HT].

The material known as Cu-Ce-hydrotalcite [Cu-Ce-HT] (Cu/ Ce/ Mg/ Al) is prepared by a standard co-precipitation method using two dilutions. The first dilution contains Mg (NO₃)₂ 6H₂O, Al(NO₃)₂ 9H₂O, Cu(NO₃)₂ 5H₂O and Ce(NO₃)₂ with a molar relationship of (Al + Mg + Cu + Ce) of 3.5. The second solution contains NaOH and Na₂CO₃ in concentrations sufficient to obtain the total precipitation of aluminum, magnesium, copper and cerium in the first dilution and to fix the pH at a value of 13. Both solutions are added slowly (addition velocity = 30 ml/hour) to the same recipient while maintaining a vigorous stirring action during 4 hours. The gel thus obtained is placed in an autoclave at a self-generated pressure of 60° C during 14 hours. Later the solid is recovered by filtration, and is washed with distilled water until pH reaches 7 and the carbonate is no longer detected in the filtering process. The hydrotalcite thus obtained is calcined at 550° C in air atmosphere during 9 hours. The result is a mixed Cu/ Ce/ Mg /Al oxide. The final quantity of Cu and Ce and the Mg/Al ratio in the solid are determined by atomic absorption measurements. The percentage by weight of the Cu obtained for the Cu-Ce-hydrotalcite material pertaining to this example is 1.5.

### Example 4: Preparation of a Cu containing polyethylenglycol (PEG) type polymeric material. Cu-PEG.

To a 0.25 (2 mmol) dilution of (CH₃COO)₂Cu in dimethylformamide (DMF) 5 g of polyethylenglycol are added. The mixture is vigorously stirred at 80° C during 24 hours. After cooling it, the resulting light brown resin is recovered by filtration, washed with DMF, water, ethanol and dichloromethane and then dried in a vacuum at ambient temperature during several hours. The metal content in the polymeric material is determined by atomic absorption and is, approximately, 1.2% by weight.

### Example 5: Preparation of a Cu containing poly-4-vynil-pyridine (PVP) type polymeric material. Cu-PVP.

To a 0.25 (2 mmol) dilution of (CH₃COO)₂Cu in dimethylformamide (DMF) 5 g of poly-4-vynil-pyridine are added. The mixture is vigorously stirred at 80° C during 24 hours. After cooling it, the resulting light brown resin is recovered by filtration, washed with DMF, water, ethanol and dichloromethane and then dried in a vacuum at ambient temperature during several hours. The metal content in the polymeric material is determined by atomic absorption and is, approximately, 1.3% by weight.

### Example 6: This example is a comparison of the results obtained during the selective oxidation of diethoxymethane with Cu (II), CuCl₂ and (CH₃COO)₂Cu salts.

100 mg of one of the materials described in the heading of this example, CuCl₂ or (CH₃COO)₂Cu, are placed in a stainless steel autoclave reactor lined with Teflon and containing a magnetic bar and 3500 mg of diethoxymethane. The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100°C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product / mols of total product *100) in each case. In this manner the following results were obtained:

**Reaction Time = 24 hours**

| Catalyst | Conversion (% Mol.) ^{a} | Selectivity (% Mol.) | | | |
|---|---|---|---|---|---|
| | | Hydrolysys ^{b} | Carbonate | Mono-oxidated ^{c} | Di-oxidated ^{d} |
| CuCl₂ | 6.2 | 15.4 | 5.0 | 4.0 | 75.6 |
| (CH₃COO)₂Cu | 2.8 | 24.4 | 0.0 | 2.4 | 73.2 |

| | | | | | |
|---|---|---|---|---|---|
| a: conversion of the maximum possible based on the initial mols of oxygen in the reaction mixture, b: acetal hydrolysis products; c: CH₃- CO - O - CH₂ - O- CH₂ -CH₃; d: CH₃ - CO - O - CH₂ - O - CO- CH₃ | | | | | |

### Example 7: This example is a comparison of the results obtained during the selective oxidation of diethoxymethane with Co (II), CoCl₂ and (CH₃COO)₂Co salts.

100 mg of one of the materials described in the heading of this example, CuCl₂ or (CH₃COO)₂Cu, are placed in a stainless steel autoclave reactor lined with Teflon and containing a magnetic bar and 3500 mg of diethoxymethane. The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 ° C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product / mols of total product * 100) in each case. In this manner the following results were obtained:

**Reaction Time = 24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| CoCl₂ | 3.6 | 30.8 | 2.6 | 2.0 | 64.8 |
| (CH₃COO)₂CO | 7.4 | 40.3 | 19.8 | 10.4 | 29.5 |

| | | | | | |
|---|---|---|---|---|---|
| a: conversion of the maximum possible based on the initial mols of oxygen in the reaction mixture; b: acetal hydrolysis products; c: CH₃- CO - O- CH₂- O- CH₂- CH₃, d: CH₃- CO - O- CH₂- 0 - CO- CH₃ | | | | | |

### Example 8: This example Is a comparison of the results obtained during the oxidation with Cu(II)Cl₂ with AIBN (2,2'-azobisiso-butyronitril) as activating agent and with AIBN in basic media.

100 mg of CuCl₂ are placed in a stainless steel autoclave reactor lined with Teflon and containing a magnetic bar and 3500 mg of diethoxymethane and 175 mg of azobisiso-butyronitril (AIBN), and in a particular case 150 mg of sodium acetate (CH₃COONa) to obtain a basic medium (pH > 9). The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 °C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant- mols of final reactant/ mols of initial reactant *100), and the selectivity to the obtained products (mols of initial product / mols of total product *100) in each case. In this manner the following results were obtained:

**Reaction Time = 24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| CuCl₂ + AIBN | 22.2 | 6.4 | 10.8 | 54.8 | 28.0 |
| CuCl₂ + AIBN (basic media) (basic media) | 45.7 | 18.6 | 11.8 | 29.3 | 7.3 |

| | | | | | |
|---|---|---|---|---|---|
| a: conversion of the maximum possible based on the initial mols of oxygen in the reaction mixture; b: acetal hydrolysis products; c: CH₃- CO - O- CH₂- O- CH₂- CH₃; d: CH₃- CO- O- CH₂- O- CO- CH₃ | | | | | |

### Example 9: This example is a comparison of the results obtained during the selective oxidation of diethoxymethane with Cu (II) Cl₂ with TBHP (tertbutyl hydroperoxide) as activating agent and with TBHP in basic media.

100 mg of CuCl₂ are placed in a stainless steel autoclave reactor lined with Teflon and containing a magnetic bar and 3500 mg of diethoxymethane and 175 mg of tertbutyl hydroperoxide (TBHP solution at 80% by weight in ditertbutylperoxide: water, 3:2), and in a particular case 150 mg of sodium acetate (CH₃COONa) to obtain a basic medium (pH> 9). The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100°C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant * 100), and the selectivity to the obtained products (mols of initial product / mols of total product* 100) in each case. In this manner the following results were obtained.

**Reaction Time = 24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| CuCl₂ + TBHP | 29.4 | 10.7 | 41.2 | 44.8 | 3.3 |
| CuCl₂ + TBHP (basic media) | 50.1 | 14.0 | 40.5 | 38.5 | 7.0 |

| | | | | | |
|---|---|---|---|---|---|
| a: conversion of the maximum possible based on the initial mols of oxygen in the reaction mixture; b: acetal hydrolysis products; c: CH₃- CO - O- CH₂- O- CH₂- CH₃; d: CH₃- CO- O- CH₂- O- CO- CH₃ | | | | | |

### Example 10: This example compares the catalytic activity shown for the selective oxidation of diethoxymethane of the materials described in Examples 1, 2 and 3.

100 mg of one of the materials described in examples 1 to 3 are placed in a stainless steel autoclave reactor lined with Teflon and containing a magnetic bar and 3500 mg of diethoxymethane. The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 ° C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product / mols of total product* 100) in each case. In this manner the following results were obtained:

**Reaction Time = 24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| Cu-HT (Ej. 1) | 5.2 | 27.0 | 24.8 | 43.0 | 5.2 |
| Co-HT (Ej. 2) | 4.5 | 37.6 | 34.6 | 19.5 | 8.3 |
| Cu-Ce-HT (Ej. 3) | 2.1 | 32.7 | 26.5 | 29.6 | 11.2 |

| | | | | | |
|---|---|---|---|---|---|
| a: conversion of the maximum possible based on the initial mols of oxygen in the reaction mixture; b: acetal hydrolysis products; c: CH₃- CO - O- CH₂- O- CH₂- CH₃; d: CH₃- CO- O- CH₂- O- CO- CH₃ | | | | | |

### Example 11: This example compares the catalytic activity shown for the selective oxidation of diethoxymethane of the materials described in Examples 1 and 2 (using an activating agent).

100 mg of one of the materials described in examples 1 and 2 are placed in a stainless steel autoclave reactor lined with Teflon and containing a magnetic bar and 3500 mg of diethoxymethane and 175 mg of activating agent that may be azobisisobutyronytril (AIBN) or tertbutyl hydroperoxide (TBHP solution at 80% by weight in ditertbutylperoxide: water, 3:2). The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 ° C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product /mols of total product * 100) in each case. In this manner the following results were obtained:

**Reaction Time = 24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| Cu-HT + AIBN | 13..0 | 27.0 | 24.8 | 43.0 | 5.2 |
| Cu-HT + TBHP | 20.1 | 12.3 | 32.9 | 41.5 | 5.4 |
| Co-HT + AIBN | 13.9 | 20.9 | 37.1 | 35.2 | 6.8 |
| Co-HT + TBHP | 20.0 | 10.2 | 32.0 | 48.6 | 9.2 |

| | | | | | |
|---|---|---|---|---|---|
| a : conversion of the maximum possible based on the initial mols of oxygen in the reaction mixture; b: acetal hydrolysis products; c: CH₃ - CO - O - CH₂ - O - CH₂-CH₃; d: CH₃-CO-O-CH₂-O-CO-CH₃ | | | | | |

### Example 12: This example compares the catalytic activity shown for the selective oxidation of diethoxymethane of the materials described in Examples 1 and 2 (using an activating agent and a basic medium).

100 mg of one of the materials described in examples 1 and 2 are placed in a stainless steel autoclave reactor lined with Teflon and containing a magnetic bar and 3500 mg of diethoxymethane and 175 mg of activating agent that may be azobisisobutyronytril (AIBN) or tertbutyl hydroperoxide (TBHP solution at 80% by weight in ditertbutylperoxide: water, 3:2). In addition, 150 mg of sodium acetate (CH₃COONa) are added to obtain a basic medium (pH > 9). The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 ° C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product / mols of total product * 100) in each case. In this manner the following results were obtained:

**Reaction Time = 24 hours**

| Catalyst | Conversion (% mol.)^{a} | Selectivity (% Mol.) | | | |
|---|---|---|---|---|---|
| | | Hydrolysis | Carbonate | Mono-oxidated | Di-oxidated |
| Cu-HT + AIBN | 34.6 | 7.2 | 41.0 | 40.0 | 11.8 |
| Cu-HT + TBHP | 37.5 | 9.5 | 43.3 | 42.2 | 5.0 |

| | | | | | |
|---|---|---|---|---|---|
| a: conversion of the maximum possible based on the initial mols of oxygen in the reaction mixture; b: acetal hydrolysis products; c: CH₃ - CO - O - CH₂ - O - CH₂ - CH₃; d: CH₃ - CO - O - CH₂ - O - CO- CH₃ | | | | | |

### Example 13: This example compares the catalytic activity shown for the selective oxidation of diethoxymethane of the materials described in Examples 4 and 5 (using an activating agent and a basic medium).

100 mg of one of the materials described in examples 4 and 5 are placed in a stainless steel autoclave reactor lined with Teflon and containing a magnetic bar and 3500 mg of diethoxymethane and 175 mg of tertbutyl hydroperoxide (TBHP solution at 80% by weight in ditertbutylperoxide: water, 3:2) used as activating agent, and 150 mg of sodium acetate (CH₃COONa) that are added to obtain a basic medium (pH > 9). The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 °C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product / mols of total product * 100) in each case. In this manner the following results were obtained:

**Reaction Time =24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| Cu-PEG (Ej. 4) | 1.7 | 29.1 | 8.2 | 52.2 | 10.5 |
| Cu-PVP (Ej. 5) | 4.9 | 57.3 | 12.9 | 10.9 | 18.9 |

| | | | | | |
|---|---|---|---|---|---|
| a: conversion of the maximum possible based on the initial mols of oxygen in the reaction mixture; b: acetal hydrolysis products; c: CH₃- CO - O- CH₂- O- CH₂- CH₃; d: CH₃- CO - O - CH₂- O - CO- CH₃ | | | | | |

### Example 14: This example compares the catalytic activity shown for the selective oxidation of diethoxymethane of the catalysts Cu (II) Cl₂ and Cu-Hydrotalcite (using an activating agent and a basic medium).

100 mg of one of the materials described in the heading of this example (Cu(II)Cl₂ or Cu-Hydrotalcite [Cu-HT] are placed in a stainless steel autoclave reactor lined with Teflon and containing a magnetic bar and 3500 mg of diethoxymethane and 175 mg of tertbutyl hydroperoxide (TBHP solution at 80% by weight in ditertbutylperoxide: water, 3:2) used as activating agent. In addition, 150 mg of sodium acetate (CH₃COONa) are added to obtain a basic medium (pH > 9). The autoclave is then hermetically closed, the lid having a connection to a pressure meter (manometer), another connection to the gaseous oxygen source and a third outlet that allows taking samples at different time intervals. The reactor is then pressurized at 18 bars with oxygen and the reaction temperature is raised to 100 °C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100). and the selectivity to the obtained products (mols of initial product / mols of total product *100) in each case. In this manner the following results were obtained:

**Reaction Time = 24 hours**

| Catalyst | Conversion (% Mol.)^{a} | Selectivity (% Mol.) | | | |
|---|---|---|---|---|---|
| | | Hydrolysis ^{b} | Carbonate | Mono-oxidated ^{c} | Di-oxidated ^{d} |
| CuCl₂ | 5.2 | 11.5 | 37.4 | 30.0 | 21.1 |
| Cu-HT | 4.0 | 24.9 | 31.3 | 21.9 | 21.9 |

| | | | | | |
|---|---|---|---|---|---|
| a: conversion of the maximum possible based on the initial mols of oxygen in the reaction mixture; b: acetal hydrolysis products; c: CH₃-CO-O-CH₂-O- CH₂-CH₃; d: CH₃-CO-O-CH₂-O-CO-CH₃ | | | | | |

### Example 15: This example compares the catalytic activity shown for the selective oxidation of 1,3 - dioxolane of the catalysts Co (II) -(OOCH₃)₂, Co-Hydrotalcite and Cu-Ce-Hydrotalcite (using an activating agent).

100 mg of one of the materials described in the heading of this example (Co(OOCH₃)₂, Co-Hydrotalcite [Co-HT], or Cu-Ce-Hydrotalcite [Cu-Ce-HT] are placed in a stainless steel autoclave reactor lined with Teflon and containing a magnetic bar and 3500 mg of 1,3-dioxolane and 40 mg of dibenzoil peroxide (DBP) used as activating agent. The autoclave is then hermetically closed, the lid having a connection to a pressure meter (manometer), another connection to the gaseous oxygen source and a third outlet that allows taking samples at different time intervals. The reactor is then pressurized at 12 bars with oxygen and the reaction temperature is raised to 100 °C. The reaction mixture is then stirred and samples are taken at various time intervals up to 24 hours of reaction. The samples are analyzed by Gas Chromatography with a FID detector, calculating the organic acetal conversion based on the composition of the mixture obtained (Mols of initial reactant - mols of final reactant/ mols of initial reactant *100), and the selectivity to the obtained products (mols of initial product / mols of total product *100) in each case. In this manner the following results were obtained:

**Reaction Time = 5 hours**

| Catalyst | Convervion (% Mol.)^{a} | Selectivity (% Mol.) Di-oxidated | | | |
|---|---|---|---|---|---|
| | | Hydrolysis ^{b} | Carbonate | Mono-oxidated ^{c} | Di-oxidated ^{d} |
| Co(OOCH₃)₂ | 53.3 | 32.0 | 11.0 | 44.0 | 13.0 |
| Co-HT | 15.6 | 11.0 | 24.0 | 62.0 | 3.0 |
| Co-HT^{e} | 39.7 | 10.0 | 24.0 | 65.0 | 1.0 |
| Cu-Ce-HT | 28.2 | 18.0 | 26.0 | 53.0 | 3.0 |

| | | | | | |
|---|---|---|---|---|---|
| a: conversion of the maximum possible based on the initial mols of oxygen in the reaction mixture; b: acetal hydrolysis products and opening and oxidation products; c: O=CH - O - CH₂- CH₂- OH; d: O=CH - O - CH₂- HC=O; c: At 60° C and 18 bars of pressure | | | | | |

## Claims

1. Procedure to obtain polyoxygenated organic compounds **characterised in that** it comprises an oxidation reaction of a diether with an oxygen source in the presence of:
a. one or more radical initiating agents,
b. one or more additives that generate an alkaline reaction medium, and
c. one or more catalysts.

2. Procedure according to claim 1, **characterized in that** the pulyoxygenated compounds are selected amongst organic carbonates, alkoxy ethers, alkoxy diethers, alkoxy esters and alkoxy diesters.

3. Procedure according to claims 1 or 2, **characterized in that** the organic polyoxygenated compounds are organic carbonates with the following formula:
R₁- O- CO- O- R₂
where R₁ and R₂ are the same or different substituents and are indistinctively selected between an alkyl with 1 to 12 carbon atoms, linear or branched, substituted or non-substituted; cyclic alkyl with 4 to 12 carbon atoms, substituted or non-substituted; and an aryl with 6 to 18 carbon atoms, substituted or non-substituted.

4. Procedure according to claim 1, **characterized in that** the polyoxygenated compounds have the following formula:
R₁-CO-O-CH₂-O-R₂
where R₁ and R₂, are the same or different substituents and have been indistinctively selected between an alkyl with 1 to 12 carbon atoms, linear or branched, substituted or non-substituted; cyclic alkyl with 4 to 12 carbon atoms, substituted or non-substituted, and an aryl with 6 to 18 carbon atoms, substiluled or non-substituted.

5. Procedure according to claim 1, **characterized in that** the organic polyoxygenated compounds have the following formula:
R₁-CO-O-CH₂-O-CO-R₂
where R₁ and R₂, are the same or different substituents and have been indistinctively selected between an alkyl with 1 to 12 carbon atoms, linear or branched, substituted or non-substituted; cyclic alkyl with 4 to 12 carbon atoms, substituted or non-substituted; or an aryl with 6 to 18 carbon atoms, substituted or non-substituted.

6. Procedure according to claim 1, **characterized in that** the polyoxygenzitod compounds obtained have an oxygen content between 20 and 70% by weight.

7. Procedure according to claim 1, **characterized in that** the polyoxygenated compounds obtained have an oxygen content between 30 and 60% by weight.

8. Procedure according to claim 1, **characterized in that** the diether is one or more organic acetals.

9. Procedure according to claim 8, **characterized in that** the organic acetal is selected between one or more linear aliphatic acetals, branched aliphatic acetals, cyclic acetals, aromatic acetals and combinations thereof.

10. Procedure according to claim 9, **characterized in that** the organic acetal has the following formula:
R₁-O-CH₂-O-R₂
where R₁ and R₂ are the same or different substituents and have been indistinctively selected between an alkyl with 1 to 12 carbon atoms, linear or branched, substituted or non-substituted; a cyclic alkyl with 4 to 12 carbon atoms, substituted or non-substituted; and an aryl with 6 to 18 carbon atoms, substituted or non-substituted.

11. Procedure according to claim 1, **characterized in that** the radical reaction initiating agent is selected between an organic nitrile, organic peroxide, an organic hydroperoxide, an inorganic hydroperoxide, and an inorganic peroxide.

12. Procedure according to claim 11, **characterized in that** the radical reaction initiating agent is an organic compound that has, at least, on nitrile group in its composition and has the following formula:
R₁-C ≡ N
where R₁ has been selected from an alkyl group with 1 to 12 carbon atoms, linear or branched, substituted or non-substituted; a cyclic alkyl group with 4 to 12 carbon atoms, substituted or non-substituted; and an aryl group with 6 to 18 carbon atoms, substituted or non-substituted; an alkylazo group with 1 to 12 carbon atoms, linear or branched, substituted or non-substituted; a bis-alkylazo group with 1 to 12 carbon atoms, linear or branched, substituted or non-substifuled; an arylazo group with 6 to 18 carbon atoms, substituted or non-substituted; a bis-arylazo group with 6 to 18 carbon atoms, substituted or non-substituted.

13. Procedure according to claim 11, **characterized in that** the radical reaction initiating agent is an organic peroxide with the following formula:
R₁-CO-O-R₂
where R₁ and R₂, are the same or different substituents and have been indistinctively selected between an alkyl with 1 to 20 carbon atoms, linear or branched, substituted or non-substituted; a cyclic alkyl with 4 to 12 carbon atoms, substituted or non-substituted; and an aryl with 6 to 18 carbon atoms, substituted or non-substituted,

14. Procedure according to claim 11, **characterized in that** the radical reaction initiating agent is an organic hydroperoxide having the following formula:
R₁-C-O-O-H
where R₁ represents a substituent selected between an alkyl with 1 to 20 carbon atoms, linear or branched, substituted or non-substituted; a cyclic alkyl with 4 to 12 carbon atoms, substituted or non-substituted; or an aryl with 6 to 18 carbon atoms, substituted or non-substituted.

15. Procedure according to claim 1, **characterized in that** said additive is one or more basic salts comprising ions selected between alkaline ions, alkali earth ions, transition metal ions and combinations thereof.

16. Procedure according to claim 15, **characterized in that** the basic salt comprises, also, one or more inorganic anions selected from carbonates, phosphates, sulphates, nitrates, chlorates, bromates, phosphonates, sulphonates, phosphites, sulphites, nitrites, chlorites, bromites and combinations thereof.

17. Procedure according to claim 15, **characterized in that** the basic salt comprises, also, one or more organic anions selected from formiates, acetates, butyrates, adipates, oxalates, phtalates, terphtalates, carbonates and combinations thereof.

18. Procedure according to claim 1, **characterized in that** the oxygen source is selected from molecular oxygen, air and a gaseous mix comprising oxygen and combinations thereof.

19. Procedure according to claim 18, **characterized in that** the oxygen source is a gaseous mixture comprising oxygen, selected from oxygen enriched air, oxygen enriched ozone, and a mixture containing nitrogen, argon and oxygen.

20. Procedure according to claim 1, **characterized in that** the catalyst is, at the least, a transition metal salt.

21. Procedure according to claim 20, **characterized in that** said transition metal is selected between one or more transition metals belonging to the groups Ib, IIb, IVb, Vb, VIb, VIIb and VIII from the periodic table.

22. Procedure according to claim 20, **characterized in that** said transition metal is selected from Mn, Ni, Fe, Cu, Co, Ce and combinations thereof.

23. Procedure according to claim 1, **characterized in that** the catalyst is a metal complex that comprises one or more organic ligands coordinated to, at least, one transition metal.

24. Procedure according to claim 23, **characterized in that** said transition metal is selected from one or more transition metals belonging to the Ib, IIb, IVb, Vb, VIb, VIIb and VIII groups from the periodic table.

25. Procedure according to claim 23, **characterized in that** said transition metal is selected from Mn, Ni, Fe, Cu, Co, Ce and combinations thereof.

26. Procedure according to claim 23, **characterized in that** said one or more organic ligands are selected from amines, alkylamines, dialkylamines, trialkylamines, arylamines, diarylamines, triarylamines, diamines, pyridines, substituted pyridines, pyrrolidines, substituted pyrrolidines, bipyridines, imines, pyrrols, substituted pyrrols, imidiazoles, substituted imidiazoles, porphyrines, phtalocyanines, and combinations thereof.

27. Procedure according to claim 1, **characterized in that** the catalyst is a compound selected between a transition metal salt and a transition metal complex, said compound being supported over a matrix selected from an amorphous solid, a microporous molecular sieve, a mesoporous molecular sieve and combinations thereof.

28. Procedure according to claim 27, **characterized in that** said amorphous solid is selected from silicon, alumina-ceria, silica-alumina, silica-ceria, a mixed transition metal oxide and combinations thereof.

29. Procedure according to claim 27, **characterized in that** said microporous molecular sieve is selected from a zeolite, clay, a pillared clay and combinations thereof.

30. Procedure according to claim 27, **characterized in that** said mesoporous molecular sieve is selected from silicate, metal-silicate, a mesoporous material obtained from the delamination of a laminar zeolitic precursor and combinations thereof.

31. Procedure according to claim 27, **characterized in that** said transition metal is selected from one or more transition metals belonging to the Ib, IIb, IVb, Vb, VIb, VIIb and VIII groups from the periodic table.

32. Procedure according to claim 31, **characterized in that** said transition metal is selected from Cu, Co, Mn, Ni, Fe, Zn, Ce and combinations thereof.

33. Procedure according to claim 1, **characterized in that** the catalyst is a hydrotalcite type solid with the following formula:
[M^{II} ₁₋ₓ M^{III}ₓ (OH) ₂] [Aⁿ⁻]_{x/n} . zH₂O
in which:
Aⁿ⁻ is one or more charge compensation ions,
n is the negative charge of the charge compensation anion,
M^{II} is one or more divalent metals,
M^{III} is one or more trivalent metals,
z varies between 1 and 10.

34. Procedure according to claim 33, **characterized in that** said charge compensation anion is selected between one or more mono-, di- and trivalent anions and combinations thereof.

35. Procedure according to claim 1, **characterized in that** the oxidation reaction is carried out in a reactor selected between a discontinuous reactor, a CSTR reactor, a fixed bed continuous reactor, a fluidized bed reactor and an ebullient bed reactor.

36. Procedure according to claim 1, **characterized in that** the oxidation reaction is carried out in a discontinuous reactor, with a diether to catalyst weight ratio between 1 and 1000.

37. Procedure according to claim 1, **characterized in that** said weight relationship between diether and catalyst is between 10 and 500.

38. Procedure according to claim 1, **characterized in that** the oxidation reaction is carried out in a discontinuous reactor, with a diether to oxidant weight ratio between 3 and 600.

39. Procedure according to claim 1, **characterized in that** the oxidation reaction is carried out in a discontinuous reactor, with a diether to initiating agent weight ratio between 1 and 500.

40. Procedure according to claim 1, **characterized in that** the oxidation reaction is carried out in a discontinuous reactor, with a diether to basic salt weight ratio between 1 and 500.

41. Procedure according to claim 1, **characterized in that** the oxidation reaction is carried out in a discontinuous reactor, with a pH range from 7 to 12.

42. Procedure according to claim 1, **characterized in that** the oxidation reaction is carried out in a discontinuous reactor, at a temperature set between 10 and 250° C.

43. Procedure according to claim 42, **characterized in that** the temperature is between 40 and 150° C.

44. Procedure according to claim 1, **characterized in that** the oxidation reaction is carried out in a discontinuous reactor, with a reaction time between 2 minutes and 72 hours.

45. Procedure according to claim 1, **characterized in that** the oxidation reaction is carried out in a discontinuous reactor at a total pressure in the system between atmospheric pressure and 100 bars.

46. Procedure according to claim 1, **characterized in that** the water generated during the oxidation reaction is separated by a stage selected from among a filtration stage involving membranes, addition of specific adsorbents and addition of compounds capable to react with the water generated during the reaction.
